# EUROPÄISCHE PATENTSCHRIFT

(11) **EP 0 996 478 B1**
(45) Veröffentlichungstag und Bekanntmachung des Hinweises auf die Patenterteilung: **12.09.2001**
(21) Anmeldenummer: 98940147.6
(22) Anmeldetag: 06.07.1998
(51) Int. Cl.: A61M 15/00, B65D 51/16, B05B 11/00

(54) **SPRAYMECHANISMUS FÜR DOSIER-SPRAYFLASCHEN**
SPRAY DEVICE FOR DOSE SPRAYING DISPENSERS
DISPOSITIF PULVERISATEUR POUR FLACONS PULVERISATEURS DOSEURS

(30) Priorität: 08.07.1997 DE 19729117
(43) Veröffentlichungstag der Anmeldung: 03.05.2000
(73) Patentinhaber: Wunsch, Erich, 75378 Bad Liebenzell (DE)
(72) Erfinder: Wunsch, Erich, 75378 Bad Liebenzell (DE)
(74) Vertreter: Söltenfuss, Dirk Christian, Dipl.-Phys.
(86) Internationale Anmeldenummer: EP9804160
(87) Internationale Veröffentlichungsnummer: WO9902211

(56) Entgegenhaltungen:
- WO-A-95/00195
- DE-C- 925 216
- GB-A- 2 095 103
- US-A- 3 838 686
- US-A- 4 072 252
- US-A- 4 694 976
- US-A- 4 730 744
- US-A- 5 273 189
- US-A- 5 524 680

## Beschreibung

Die Erfindung betrifft einen Spraymechanismus für Dosier-Sprayflaschen zur Feinzerstäubung von Flüssigkeiten, und insbesondere für Sprayflaschen zur Feinzerstäbung von pharmazeutischen Flüssigkeiten zur Behandlung von Krankheiten der Atemwege, wie beispielsweise Asthma bzw. Bronchialasthma, nach dem Oberbegriff von Anspruch 1.

Es sind bereits Dosier-Spraysysteme der eingangs genannten Art bekannt und im Handel, bei denen durch Fingerdruck mechanisch zu betätigende, hydraulisch wirkende Pumpen zum Feinzerstäuben von Flüssigkeiten in Sprayflaschen oder Spraydosen eingesetzt werden.

Aus "Medizinische Klinik", 90 (1995), 617 (Nr. 11), Verlag Urban & Vogel, München, Seite 23, geht hervor, daß bei den eingesetzten Dosier-Spraysystemen als Treibgas eine Mischung verschiedener Fluorchlorkohlenwasserstoffe (FCKW) verwendet wird. Von den FCKW ist bekannt, daß sie die Ozonschicht zerstören. Die Vereinten Nationen vereinbarten deshalb im Protokoll von Montreal, die Produktion von FCKW bis zum 01.01.96 einzustellen. Da die medizinische Anwendung in Dosieraerosolen zur Behandlung obstruktiver Atemwegserkrankungen zur Zeit unentbehrlich ist, wurde für FCKW eine Ausnahmegenehmigung für medizinische Zwecke über 1996 hinaus erteilt.

Neben den Dosieraerosolen stehen Pulverinhalationssysteme und Vernebelungsgeräte zur Verfügung, die aber alle Nachteile haben, wie beispielsweise das Verklumpen von Pulver bei Feuchtigkeit.

Als alternative Treibgase wurden inzwischen Hydrofluoralkane entwickelt. Diese Substanzen sind brennbar, enthalten kein Chlor und besitzen demzufolge keine die Ozonschicht zerstörende Wirkung. Wie viele andere gasförmige Substanzen in der Athmospäre auch tragen diese neuen Treibgase aber zum sogenannten Treibhauseffekt bei.

Aus der deutschen Patentschrift DE C1 31 22 682 ist beispielsweise ein Ultraschallinhalator bekannt, der einen auswechselbaren Inhalatbehälter und eine Fördereinrichtung für das Inhalat vom Inhalatbehälter zum Resonatorkopf aufweist. Die Inhalatförderung erfolgt bei dem in dieser Druckschrift offenbarten Pumpmechanismus über eine von einem Motor angetriebene Gewindespindel mit daran befestigtem Druckteller, der gegen die Bodenfläche des verformbaren Inhalatbehälters gedrückt wird. Hierdurch wird ein Ultraschallinhalator für unterschiedliche Anwendungszwecke geschaffen, bei dem trotz unterschiedlicher Zusammensetzung der Inhalate stets die gleiche Menge des Inhalates der Resonatorkopfplatte zugeführt wird.

Ferner wird in der deutschen Patentschrift DE C1 39 11 985 ein Inhaliergerät für Dosier-Aerosole beschrieben. Dieses Inhaliergerät weist ein gegenüber dem Flüssigkeitsbehälter in eine abgewinkelte Gebrauchsstellung verschwenkbares Sprührohr auf, das zu einem Mundstück führt. Die Betätigung des Inhaliergerätes erfolgt durch zwei aufeinander gegenüberliegenden Seiten am Flüssigkeitsbehälter angelenkte Betätigungsorgane, die zwischen Daumen und Zeigefinger quer zur Behälterachse gegneinander gepreßt werden und dabei über Keilflächen einen axialen Betätigungsschub des Flüssigkeitsbehälters auslösen.

Bei diesen und anderen bekannten Systemen setzt der Sprühvorgang immer erst dann ein, wenn der in der Kammer der Pumpe erzeugte Flüssigkeitsdruck ausreichend hoch ist und beispielsweise einen Wert von etwa 6 bis 8 bar überschreitet. Aus diesem Grunde ist es nicht möglich, jederzeit bei Betätigen des Spraymechanismus unverzüglich einen stetigen Sprühvorgang zu beginnen.

Insbesondere bei einer akuten Behandlung von Erkrankungen der Atemwege, wie beispielsweise der Behandlung eines Asthmaanfalles, kann es für den Patienten unter Umständen lebenswichtig sein, die notwendigen Pharmazeutika sofort und ohne zeitliche Verzögerung inhalieren zu können. Denn die Pharmazeutika wirken für den Patienten nur in der Anfangsphase eines Asthmaanfalles. Zu diesem Zweck werden im Handel üblicherweise Sprayflaschen im Taschenformat angeboten, die der Patient ständig bei sich tragen kann.

Aus dem europäischen Patent EP B1 0 365 753 des Anmelders ist bereits ein Spraymechanismus für andere Anwendungsfälle bekannt, bei dem ohne Umweltbelastung ein zumindest nahezu ununterbrochener Sprühstrahl aus der Zerstäuberdüse der Sprayflasche austritt. Der Antrieb der Pumpe erfolgt bei diesem System elektromotorisch durch einen Getriebemotor und einen Exzenter, der durch eine oder mehrere Schlingfedern mitgenommen wird. Ferner ist es aus dieser Druckschrift bekannt, ein beispielsweise federbelastetes Luftventil vorzusehen, das mit einem in den Aufnahmebehälter der Sprayflüssigkeit hineinragenden Luftrohr verbunden ist und über einen Elektroschalter des Getriebemotors bedienbar ist.

Die oben beschriebenen, bekannten Ventilanordnungen haben jedoch einen Nachteil, daß sie den Austritt der Sprayflüssigkeit aus dem Aufnahmebehälter nicht unter allen üblicherweise auftretenden Bedingungen verhindern können.

Vor allem die oben genannten Sprayflaschen im Taschenformat für die Behandlung von Erkrankungen der Atemwege werden vom Patienten überallhin mitgenommen. Die Sprayflaschen befinden sich also insbesondere nicht immer aufrecht und können auch unterschiedlichen Druckbedingungen ausgesetzt sein, wie beispielsweise im Flugzeug. Für den Patienten ist es in jedem Fall unerläßlich, bei Bedarf eine funktionsfähige Sprayflasche mit ausreichend pharmazeutischer Sprayflüssigkeit zur Hand zu haben, weshalb ein unerwünschtes Austreten auch kleinster Mengen der Sprayflüssigkeit aus dem Aufnahmebehälter unbedingt vermieden werden muß.

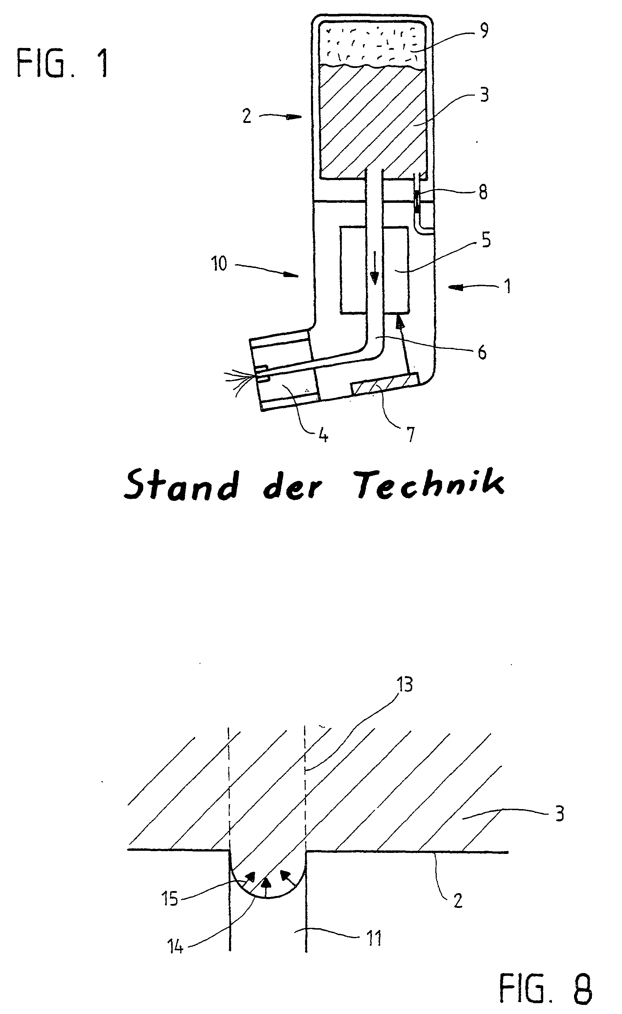

Ein weiterer Spraymechanismus für Dosier-Sprayflaschen ist zum Beispiel aus der US-A-4,072,252 bekannt, welche sämtliche Merkmale des Oberbegriffs von Anspruch 1 offenbart. Insbesondere umfaßt dieser Spraymechanismus einen Aufnahmebehälter zur Aufnahme von Flüssigkeiten, eine Zerstäuberdüse zur Abgabe der Flüssigkeit aus einer Sprayflasche, einen Pumpmechanismus zum Pumpen der Flüssigkeit aus dem Aufnahmebehälter durch einen Medienkanal zu der und durch die Zerstäuberdüse nach außen und eine Betätigungsvorrichtung zum Betätigen des Pumpmechanismus. Weiter ist eine Ventilanordnung vorgesehen, die eine Luftzufuhr von außen in den Aufnahmebehälter bei Betätigung des Pumpmechanismus ermöglicht, wobei ein Austritt von Flüssigkeit durch die Ventilanordnung aus dem Aufnahmebehälter im wesentlichen verhindert ist. Diese Ventilanordnung umfaßt dabei insbesondere eine Bohrung von kleinem Durchmesser, die aufgrund ihrer Kapillarwirkung die oben beschriebene Ventilwirkung erzielt. Das Grundprinzip eines derartigen Spraymechanismus ist beispielsweise auch in Fig. 1 dargestellt, die weiter unten näher beschrieben wird.

Ausgehend von diesem Stand der Technik ist es eine Aufgabe der vorliegenden Erfindung, einen Spraymechanismus für Dosier-Sprayflaschen der eingangs genannten Art weiterzuentwickeln, der - insbesondere für Sprayflaschen, deren Gebrauchsstellung auf dem Kopf gestellt ist- bei Betätigung des Pumpmechanismus jederzeit und unverzüglich einen Sprühvorgang gewährleistet und ein unerwünschtes Austreten der Sprayflüssigkeit verhindert.

Diese Aufgabe wird durch einen Spraymechanismus mit den Merkmalen des Anspruchs 1 gelöst.

Die Ventilanordnung des erfindungsgemäßen Spraymechanismus befindet sich im Bodenbereich des Aufnahmebehälters und weist eine Öffnung in Form einer Bohrung oder eines Rohres auf. Sprayflaschen z.B. für die Behandlung von Erkrankungen der Atemwege (z.B. Asthma) werden üblicherweise in einer Gebrauchsstellung eingesetzt, wie sie in Fig. 1 dargestellt ist. Bei einer derartigen Gebrauchsstellung strömt die Luft durch die erfindungsgemäße Ventilanordnung bei Betätigung des Pumpmechanismus in den Aufnahmebehälter in den Bereich, in dem sich bereits Luft befindet. Hierdurch ist das Einströmen der Luft für den Druckausgleich im Innern des Aufnahmebehälters im Vergleich zu dem Fall, bei dem die Luft in den mit Flüssigkeit gefüllten Bereich des Aufnahmebehälters eindringt, wesentlich vereinfacht, was zu einem schnelleren und sichereren Druckausgleich führt.

Ein besonderer Vorteil der erfindungsgemäßen Ausführung der Dosier-Sprayflaschen bzw. der Dosier-Sprayeinrichtungen wird auch darin gesehen, daß mit der neuen Ausführung die Handhabung für den Anwender/Patienten gleich geblieben ist und keine Umstellung im Gebrauch und in der Positionierung der Einrichtung gefordert wird.

Weitere Ausgestaltungen und Weiterbildungen der Erfindung sind Gegenstand der Unteransprüche.

Die Erfindung wird nachfolgend anhand verschiedener erläuternder Beispiel und Ausführungsbeispiele der Erfindung unter Bezugnahme auf die beilöiegende Zeichnung näher beschrieben. Darin zeigen:
- Fig. 1: in vereinfachter, schematischer Darstellung das Grundprinzip eines bekannten Spraymechanismus;
- Fig. 2 bis 6: verschiedene Beispiele von Dosier-Sprayeinrichtungen, die zum besseren Verständnis der vorliegenden Erfindung erläutert werden;
- Fig. 7A bis C: ein erstes Ausführungsbeispiel einer Sprayeinrichtung gemäß der vorliegenden Erfindung;
- Fig. 8: eine schematische Darstellung zur Erläuterung der Ventilfunktion der erfindungsgemäßen Ventilanordnung;
- Fig. 9: das Aufsatzteil der Sprayeinrichtung des in Fig. 2 gezeigten Beispieles vergrößert im Schnitt;
- Fig. 10: ein zweites Ausführungsbeispiel einer Sprayeinrichtung gemäß der vorliegenden Erfindung;
- Fig. 11A und B: ein drittes Ausführungsbeispiel einer Sprayeinrichtung gemäß der vorliegenden Erfindung; und
- Fig. 12: ausschnittsweise ein viertes Ausführungsbeispiel einer Sprayeinrichtung gemäß der vorliegenden Erfindung.

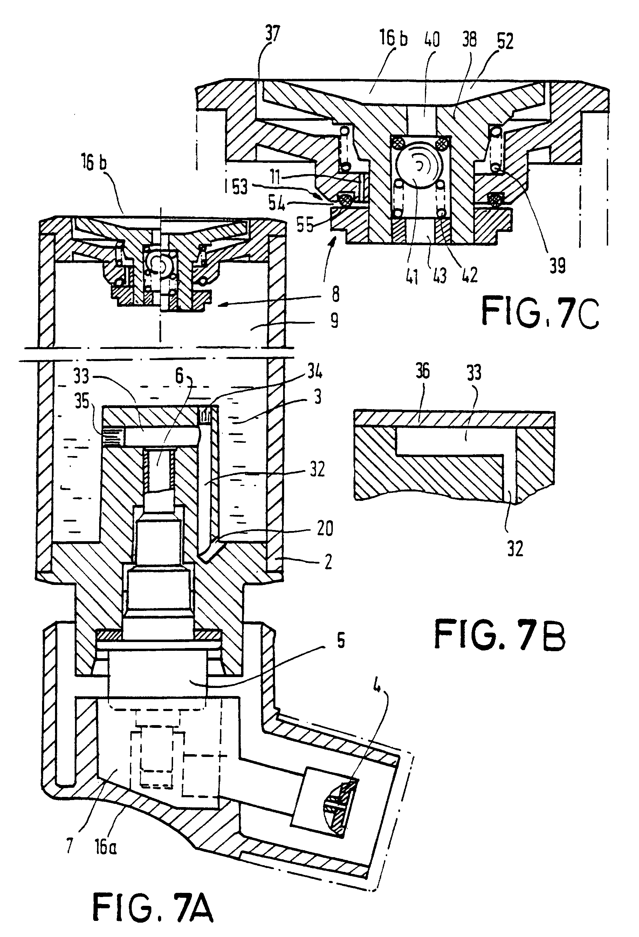

Im folgenden wird der erfindungsgemäße Spraymechanismus für die Sprayeinrichtungen zur Feinzerstäubung von Flüssigkeiten anhand von bevorzugten Ausführungsbeispielen der Erfindung (Fig. 7-12) und Verschiedenen Beispielen anderer Spraymechanismen (Fig. 1-6) beschrieben. Dabei werden in allen Figuren für gleiche Elemente die gleichen Bezugszeichen verwendet. Die Begriffe Sprayflasche oder Dosier-Sprayflasche und Sprayeinrichtung oder Dosier-Sprayeinrichtung stehen immer gleichwertig nebeneinander, wobei der Ausdruck Dosier-Sprayflasche der in der Praxis häufigere Begriff ist, weil mit ihm auf die wesentlichen Eigenschaften des dosierten Sprühens hingewiesen wird. Die Beispiele der Fig. 1-6 dienen nur dem besseren Verständnis der vorliegenden Erfindung; sie sind nicht gegenstand der Erfindung, wie sie in den anliegenden Ansprüchen definiert ist.

Zunächst ist in Figur 1 das Grundprinzip eines bekannten Spraymechanismus dargestellt. Figur 1 zeigt beispielhaft eine Sprayflasche 1 zur Behandlung von Erkrankungen der Atemwege in ihrer Gebrauchsstellung, d.h. mit dem Flüssigkeitsaustritt durch eine Zerstäuberdüse 4 nach unten gerichtet. Die Sprayflasche 1 ist etwa in einem Maßstab 1: 1 dargestellt und faßt etwa 20 bis 25 ml Sprayflüssigkeit 3.

Die Sprayflasche 1 besteht im wesentlichen aus einem Aufnahmebehälter 2, der mit der Sprayflüssigkeit 3 gefüllt ist, wobei im Inneren des Aufnahmebehälters 2 Normaldruck herrscht, und aus einem Aufsatzteil 10, welches auf den Aufnahmebehälter 2 in einer dichtenden Art und Weise aufgesetzt ist. Die Dichtverbindung zwischen Aufnahmebehälter 2 und Aufsatzteil 10 ist insbesondere bei der Verwendung von pharmazeutischen Sprayflüssigkeiten 3 eine nicht lösbare Verbindung, um sicherzustellen, daß sich auch nur die erwünschte Sprayflüssigkeit bzw. Medizin 3 in der Sprayflasche 1 befinden kann. In anderen, beispielsweise nicht-medizinischen Anwendungsfällen kann eine in an sich bekannter Weise lösbare Verbindung zwischen Aufnahmebehälter 2 und Aufsatzteil 10 vorgesehen sein, so daß dann die Möglichkeit besteht, das Aufsatzteil für mehrere Aufnahmebehälter 2 austauschbar immer wieder zu verwenden.

Das Aufsatzteil 10 weist an seinem dem Aufnahmebehälter 2 abgewandten Ende eine Zerstäuberdüse 4 für die dosierte Abgabe der Sprayflüssigkeit 3 aus der Sprayflasche 1 auf. Die Zerstäuberdüse 4 ist vorzugsweise, wie in Figur 1 gezeigt, relativ zu der Längsachse der Sprayflasche 1 in einem bestimmten Winkel angeordnet, um die Inhalation der Arzneiflüssigkeit 3 zu erleichtern. Diese Lage der Dosier-Sprayflasche 1 ist in der Praxis für Asthmaerkrankte seit langem und weltweit eingeführt.

Die Zerstäuberdüse 4 ist über einen Medienkanal 6 mit dem Inneren des Aufnahmebehälters 2 verbunden. Weiter enthält das Aufsatzteil 10 einen Pumpmechanismus 5, der bei Betätigung über ein Betätigungselement 7 die Sprayflüssigkeit 3 aus dem Aufnahmebehälter 2 durch den Medienkanal 6 zur Zerstäuberdüse 4 und durch diese hindurch nach außen pumpt. Als Pumpmechanismus 5 sind aus dem allgemeinen Stand der Technik verschiedene Systeme mechanischer, hydraulischer und elektromotorischer Art bekannt; als Betätigungselement 7 sind ebenfalls verschiedenste Ausführungen bekannt, die mit dem Pumpmechanismus 5 entsprechend wirkverbunden werden.

Weiter besitzt die Dosier-Sprayflasche 1 eine in Figur 1 nur symbolisch dargestellte Ventilanordnung 8. Die Ventilanordnung 8 verbindet den Innenraum des Aufnahmebehälters 2 mit dem Außenraum der Sprayflasche 1, d.h. mit dem üblichen Umgebungsmedium Luft. Die Ventilanordnung 8 ist erfindungsgemäß so konstruiert, daß eine Luftzufuhr von außen in den Aufnahmebehälter 2 zumindest während der Betätigung des Pumpmechanismus 5, d.h. während des Sprühvorganges möglich ist. Andererseits wird ein Austritt der Sprayflüssigkeit 3 durch die Ventilanordnung 8 aus dem Aufnahmebehälter 2 nach außen prinzipiell verhindert, d.h. sowohl zu der Zeit als auch bei jedem Außendruck und jeder Stellung der Sprayflasche 1. Hierdurch wird regelmäßig ein ständiger Druckausgleich durch die Luftmenge 9 im Aufnahmebehälter 2 gewährleistet, so daß jederzeit und unabhängig von den äußeren Bedingungen vor und während des Betätigens des Pumpmechanismus 5 ein sofortiger Sprühvorgang möglich ist.

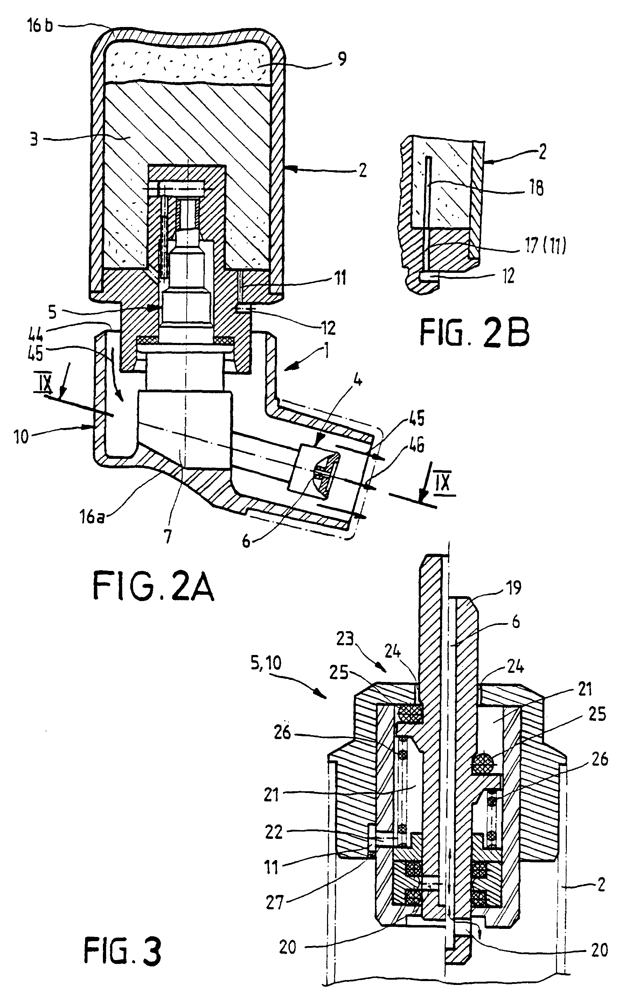

Im folgenden werden zunächst anhand der Fig. 2-6 Beispiele von Dosier-Sprayflaschen 1 näher beschrieben, die von dem in Fig. 1 dargestellten Spraymechanismus Gebrauch machen.

Zunächst wird anhand von Figur 2 der bezüglich des Aufbaus der Ventilanordnung 8 einfachste Spraymechanismus beschrieben. In diesem ersten Beispiel besteht die Ventilanordnung 8 im wesentlichen nur aus einer Bohrung 11, die den Innenraum des Aufnahmebehälters 2 mit dem Außenraum der Dosier-Sprayflasche 1 verbindet, wie dies in Figur 2A gezeigt ist. Bei der in Figur 2 gezeigten Dosier-Sprayflasche 1 ist die Bohrung 11 in dem Aufsatzteil 10 angebracht, welches auf dem Aufnahmebehälter 2 aufsitzt. Die Bohrung 11 kann aber ebenso an jeder beliebigen anderen Stelle auch am Aufnahmebehälter 2 selbst vorgesehen sein.

An dem dem Innenraum des Aufnahmebehälters 2 abgewandten Ende der Bohrung 11 ist vorteilhafterweise eine weitere Bohrung 12 vorgesehen, die die Bohrung 11 direkt mit dem Außenraum verbindet. Die Bohrachse der Bohrung 12 ist dabei in einem Winkel von etwa 90° (+ 15°) zu der Bohrachse der Bohrung 11 ausgerichtet. Der Durchmesser der zweiten Bohrung 12 ist so bemessen, daß er ein Eindringen von Partikeln in die Bohrung 11 und somit ein Verstopfen derselben verhindern kann. Zusätzlich zu oder anstelle der zweiten Bohrung 12 kann auch ein feines Gitter, ein Filter oder dergleichen angebracht werden, um die Bohrung 11 besser vor einem Verschmutzen bzw. Verstopfen zu schützen.

Um mit der Bohrung 11 die gewünschten Ventilfunktionen zu erreichen, muß die Bohrung 11 bezüglich ihres Durchmessers und ihrer Länge so bemessen sein, daß die Flüssigkeit 3 bei jeder Stellung der Sprayflasche 1 und jedem üblicherweise auftretenden Außendruck nicht durch die Bohrung 11 aus dem Aufnahmebehälter 2 heraustreten kann. Das Eindringen der Luft von außen in den Aufnahmebehälter 2 ist bei den üblicherweise herstellbaren Bohrungen 11 in jedem Fall unkritisch.

Bei der Wahl der Abmessungen der Bohrung 11 sind insbesondere die Kohäsionskräfte und die Oberflächenspannung der Flüssigkeit 3 sowie die Adhäsionskräfte zwischen der Flüssigkeit 3 und der Innenwand der Bohrung 11 zu berücksichtigen. Die Kohäsionsund Adhäsionskräfte der Flüssigkeit 3 sind verantwortlich für die zwischen der Flüssigkeit 3 und der Bohrungsinnenwand und der Luft auftretenden Grenzflächenspannungen.

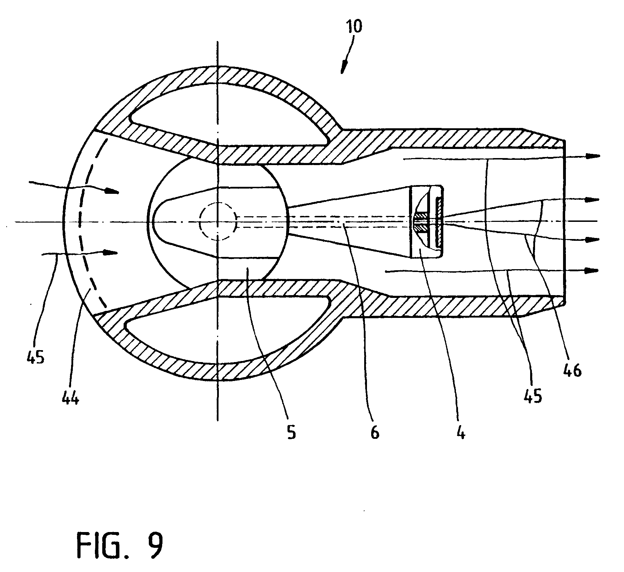

In Figur 8 sind die entsprechenden Verhältnisse für den ungünstigsten Fall dargestellt, d.h. die Bohrung 11 befindet sich in der gezeigten Stellung der Sprayflasche 1 unten an dem Aufnahmebehälter 2. In diesem Fall ist die Flüssigkeitssäule 13 über der Bohrung 11 am höchsten und übt damit auch den größtmöglichen Schweredruck aus.

Der Schweredruck der Flüssigkeit 3 ist proportional zu der Höhe der Flüssigkeitssäule 13 und zu der Dichte der Flüssigkeit 3. Die Flüssigkeitssäule 13 steht mit ihrem Gewicht auf der Randlinie der Bohrung 11. Im Gleichgewicht ist dieser Schweredruck gleich dem Zug der halbkugeligen Oberfläche 14 der in der Bohrung 11 anstehenden Flüssigkeit 3. Der Zug der Oberfläche 14, die aufgrund der Kohäsionskräfte 15 der Flüssigkeit 3 eine kugelige Form annimmt, ist proportional zu der Grenzflächenspannung und umgekehrt proportional zum Radius der Bohrung 11. Dies bedeutet, je kleiner der Radius der Bohrung 11 ist, umso größer ist der Zug der halbkugeligen Oberfläche 14 und umso mehr Flüssigkeit 3 kann über der Bohrung anstehen ohne hindurchzufließen.

Die Grenzflächenspannung ist außerdem abhängig von den einzelnen Grenzflächenspannungen zwischen der Flüssigkeit und Luft, zwischen der Flüssigkeit und der Bohrungsinnenwand und zwischen der Bohrungsinnenwand und Luft. In Figur 8 ist der Fall dargestellt, daß die Grenzflächenspannung zwischen der Flüssigkeit und der Bohrungsinnenwand positiv und größer als die Grenzflächenspannung zwischen Luft und der Bohrungsinnenwand ist, so daß die Haftspannung der Flüssigkeit an der Bohrungswand negativ wird und die Flüssigkeit die Bohrungsinnenwand nicht benetzt. Im anderen Fall, d.h. wenn die Flüssigkeit die Bohrungsinnenwand benetzt, würde die Flüssigkeit an der Bohrungsinnenwand hochkriechen, was für die hier geforderte Ventilfunktion der Bohrung 11 nicht wünschenwert ist. Aus diesem Grund kann die Innenwand der Bohrung 11 so präpariert oder das Material der Bohrungswand so gewählt werden, daß die Bohrungsinnenwand nicht oder nur minimal von der Sprayflüssigkeit 3 benetzt wird.

Weiter haben auch die Umweltbedingungen der Sprayflasche 1 einen Einfluß auf die Ventileigenschaften der Bohrung 11. Je nach herrschendem Außendruck erfährt die Sprayflüssigkeit 3 durch den Druckunterschied zwischen Innen- und Außenraum des Aufnahmebehälters 2 eine zusätzliche Sogwirkung nach außen oder eine Druckerhöhung ins Innere. Auch Temperaturschwankungen beeinflussen die Druckverhältnisse: je nach Temperatur dehnt sich die Flüssuigkeit 3 im Aufnahmebehälter 2 aus oder zieht sich zusammen, so daß sich der Druck im Inneren des Aufnahmebehälters 2 entsprechend ändert.

Die erforderlichen Abmessungen der Bohrung 11 hängen natürlich zudem von der verwendeten Sprayflüssigkeit 3 und ihren physikalischen und chemischen Eigenschaften ab. Versuche haben gezeigt, daß eine Bohrung 11 mit einem Durchmesser von höchstens etwa 0,25 mm bis etwa 0,35 mm geeignet ist, ein Austreten der zur Zeit auf dem Markt gängigen Sprayflüssigkeiten, insbesondere pharmazeutischer Art, in einem Temperaturbereich von etwa bis + 30° C zu verhindern. Als Material der Bohrungsinnenwand haben sich beispielsweise Kunststoffe, wie Plexiglas, Acrylglas, PP mit PTFE (Teflon) legiert als vorteilhaft erwiesen, wobei die Innenwand zusätzlich noch behandelt (beschichtet oder mechanisch) werden kann, um die Benetzung mit der Flüssigkeit 3 zu verringern.

Das Prinzip dieses geschilderten Spraymechanismus ist nun wie folgt:

Bei Gebrauch hält man die Dosier-Sprayflasche 1 an den entsprechenden Ausbuchtungen 16a und 16b zwischen Daumen und Zeigefinger und drückt diese zusammen. Vor der Zerstäuberdüse 4 im Inneren der Sprayflasche 1 befindet sich eine kleine Menge der Sprayflüssigkeit 3, beispielsweise etwa 0,1 ml; diese Flüssigkeitsmenge wird beim Zusammendrücken der Sprayflasche 1 und dem damit verbundenen Herunterdrücken des Kolbens des Pumpmechanismus 5 aus der Zerstäuberdüse 4 herausgedrückt. Beim Herunterdrücken des Kolbens erfolgt ein Sprühstoß (Pfeil 46). Beim automatischen Rückzug des Kolbens durch eine Feder wird sofort wieder neue Flüssigkeit aus dem Aufnahmebehälter 2 angesaugt; hierzu reicht die Schwerkraftswirkung auf die Flüssigkeit 3 und die Vergrößerung des für die Flüssigkeit 3 zur Verfügung stehenden Volumens aus. Gleichzeitig strömt durch die Bohrung 11 Luft von außen in den Aufnahmebehälter 2 nach und gewährleistet einen ständigen und sofortigen Druckausgleich im Behälter 2, d.h. in dem Aufnahmebehälter 2 entsteht durch das Nachströmen der Flüssigkeit 3 in den Medienkanal 6 kein Unterdruck, der dem Nachströmen entgegenwirken kann. Beim Loslassen des Betätigungselementes 7 bzw. beim Nachlassen des Druckes auf die Andruckfläche 16a wird der Medienkanal 6 wieder geschlossen, d.h. die Flüssigkeit 3 kann nicht mehr in den Aufnahmebehälter 2 zurückfließen, sondern steht wieder direkt an der Zerstäuberdüse 4 in exakt dosierter Menge an. Hierdurch ist es möglich, jederzeit einen sofortigen Sprühvorgang mit vorgeschriebener Dosiermenge zu starten, ohne daß zunächst ein bestimmter Druck im Behälter 2 und im Pumpmechanismus 5 aufgebaut werden muß.

Zusäztlich ist im Aufsatzteil 10 bzw. an der Verbindung Aufsatzteil 10 und Aufnahmebehälter 2 eine Lufteintrittsöffnung 44 vorgesehen, die den Innenraum des Aufsatzteiles 10 mit der Umgebung der Sprayflasche 1 verbindet. Bei Gebrauch der Dosier-Sprayflasche 1 betätigt der Patient nicht nur die Betätigungsvorrichtung 7 des Pumpmechanismus 5, sondern atmet gleichzeitig auch kräftig ein. Hierdurch wird Luft durch die Lufteintrittsöffnung 44 durch das Aufsatzteil 10 hindurch an der Zerstäuberdüse 4 vorbei zusammen mit der Sprayflüssigkeit 3 aus der Zerstäuberdüse 4 (Pfeil 46) in den Mund des Patienten gesogen (Pfeil 45).

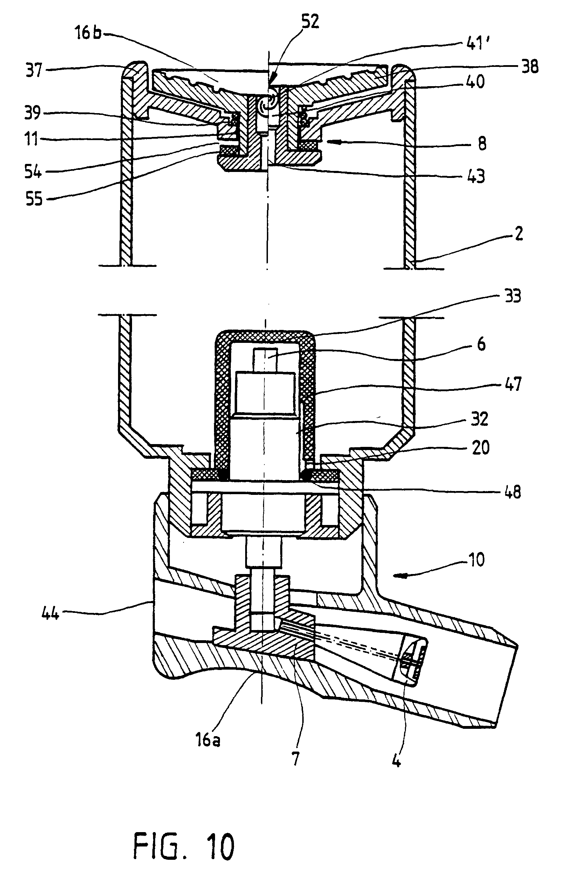

Wie in dem in Figur 9 dargestellten Schnitt des Aufsatzteiles 10 gemäß Linie IX-IX von Figur 2A gezeigt, wird die aus der Zerstäuberdüse auistretende Sprayflüssigkeeit 3, 46 von einem Luftmantel 45 umgeben. Die Lufteintrittsöffnung 44 entspricht hierbei nicht der von Figur 2A sondern der des anhand von Figur 10 weiter unten noch zu beschreibenden sechsten Ausführungsbeispieles einer erfindungsgemäßen Sprayeinrichtung. Durch diese Maßnahme wird der Wirkungsgrad eines Sprühvorganges deutlich verbessert, da die Sprühflüssigkeit 3 und damit der medizinische Wirkstoff nicht - wie bei herkönmmlichen Sprayflaschen - zu großen Anteilen im Gaumen- und Mundraum bleibt, sondern zusammen mit dem Luftmantel 45 in die Atemwege des Patienten gelangt. Der Luftmantel 45 des Sprühstrahles 46 verhindert wirksam eine zu große Ausweitung des Sprühstrahles 46. Vermutlich wird der Sprühstrahl 46 auch dadurch besser gebündelt, daß vor der Zerstäuberdüse 4 durch die angesogene Luft 45 ein Unterdruck entsteht.

Die getrennte, auszugsweise Darstellung von Figur 2B zeigt, daß die Ventilanordnung 8 nicht nur aus einer Bohrung 11, sondern ebenso aus einem in eine Öffnung 17 (oder in die Bohrung 11) paßdicht eingesetzten Rohr 18 bestehen kann, welches wie die Bohrung 11 des oben beschriebenen Ausführungsbeispiels den Innenraum des Aufnahmebehälters 2 mit dem Außenraum der Sprayflasche 1 verbindet.

Bezüglich der Abmessungen und der Eigenschaften des Rohres 18 gilt das gleiche wie für die oben beschriebene Bohrung 11. Das Rohr 18 ragt vorteilhafterweise ein gewisses (begrenztes) Stück lang in den Innenraum des Aufnahmebehälters 2 hinein. Durch diese Maßnahme kann die wirksame Länge des Rohres 18 vergrößert und die auf der Querschnittsfläche des Rohres 18 aufstehende Flüssigkeitssäule 13 verringert werden, was die Sicherheit gegen einen eventuellen Flüssigkeitsaustritt aus dem Aufnahmebehälter 2 erhöht.

In Figur 3 ist ausschnittsweise das Aufsatzteil 10 bzw. der Pumpmechanismus 5 eines zweiten Beispieles eines Spraymechanismus dargestellt. In der linken Hälfte von Figur 3 ist dabei der Ruhezustand der Ventilanordnung 8 bei Nicht-Betätigung des Pumpmechanismus gezeigt, während in der rechten Hälfte von Figur 3 der Zustand bei Betätigung des Pumpmechanismus mit geöffnetem Ventil dargestellt ist.

Der Aufnahmebehälter 2 für die Sprayflüssigkeit ist nur strichpunktiert angedeutet. In der Mitte des Pumpmechanismus 5 befindet sich der Ventilstößel 19 mit dem hohlen Medienkanal 6 in seinem Inneren. Der Ventilstößel 19 weist an seinem dem Aufnahmebehälter 2 zugewandten Ende zumindest eine Medieneintrittsöffnung 20 auf, die bei Betätigung des Pumpmechanismus 5, d.h. bei Herunterdrücken des Ventilstößels 19, den Innenraum des Aufnahmebhälters 2 mit dem Medienkanal 6 verbindet, so daß die Sprayflüssigkeit 3 in den Medienkanal 6 hineinströmen kann, wie dies deutlich durch die Pfeile in der rechten Hälfte von Figur 3 gezeigt ist. Die Medieneintrittsöffnung 20 ist dagegen im Ruhezustand, d.h. bei Nicht-Betätigung des Pumpmechanismus 5 verschlossen, so daß ein Eintritt von Flüssigkeit 3 in den Medienkanal 6 nicht möglich ist (linke Hälfte von Figur 3).

Die Dosier-Sprayflasche oder Dosier-Sprayeinrichtung 1 besitzt wie das erste Ausführungsbeispiel von Figur 2 eine Bohrung 11, die eine Luftzufuhr vom Außenraum der Dosier-Sprayflasche 1 in den Aufnahmebehälter 2 ermöglicht. Im Ausführungsbeispiel von Figur 3 ist die Bohrung 11 jedoch nicht direkt mit dem Außenraum der Dosier-Sprayflasche 1 verbunden, sondern führt über eine Luftdurchgangsöffnung 22 in eine Ventilkammer 21 in dem Aufsatzteil 10. Diese Ventilkammer 21 ist wiederum über ein Ventil 23 mit dem Außenraum der Dosier-Sprayflasche 1 verbunden. Das Ventil 23 besteht aus einer Lufteintrittsöffnung 24 und einer Dichtung 25 in Form eines vorzugsweise elastischen Dichtrings. Bei Nicht-Betätigung des Pumpmechanismus 5, d.h. im Ruhezustand der Einrichtung, wird dieser Dichtring 25 mittels einer Druckfeder 26 gegen die Lufteintrittsöffnung 24 gedrückt und verschließt die Lufteintrittsöffnung 24, so daß aus dem Außenraum der Dosier-Sprayflasche 1 keine Luft in die Ventilkammer 21 eindringen kann (linke Hälfte von Figur 3). Die Druckfeder 26 stützt sich einmal an einem Ringflansch des Ventilstößels 19 und zum anderen an einem ortsfesten Ring im Aufsatzteil 10 ab. Bei Betätigung des Pumpmechanimus 5 wird durch das Herunterdrücken des Ventilstößels 19 die Druckfeder 26 zusammengedrückt und dabei der Dichtring 25 von seiner dichten Anlage an der Lufteintrittsöffnung 24 gelöst (rechte Hälfte von Figur 3). Hierdurch wird die Luftzufuhr durch die Lufteintrittsöffnung 24 in die Ventilkammer 21 und von dieser durch die Luftdurchgangsöffnung 22 und die Bohrung 11 in den Aufnahmebehälter 2 ermöglicht. Dadurch, daß bei Betätigung des Pumpmechanismus 5 gleichzeitig mit dem Öffnen des Medienkanals 6 auch die Luftzufuhr durch die Ventilanordnung 8 bewirkt wird, wird ein sofortiger und ständiger Druckausgleich im Aufnahmebehälter 2 gewährleistet.

Durch das Vorschalten einer Ventilvorrichtung aus Ventilkammer 21 und Ventil 23 vor die Bohrung 11 ist diese Bohrung zumindest bei Nicht-Betätigung des Pumpmechanismus vom Außenraum der Dosier-Sprayflasche 1 entkoppelt und somit die Ventilfunktion vom Außendruck unabhängig.

Anstelle der Bohrung 11 kann selbstverständlich auch, wie beim ersten Beispiel gemäß Figur 2B, ein Rohr 18 zum Einsatz kommen.

Ferner kann, wie in Figur 3 angedeutet, die Bohrung 11 (bzw. das Rohr 18) mit einer Membran 27 versehen sein. Diese Membran 27 ist dabei so beschaffen, daß sie einerseits luftdurchlässig ist, aber andererseits den Durchtritt der Flüssigkeit 3 zumindest in der einen Richtung aus dem Aufnahmebehälter 2 heraus sperrt. Durch diese Maßnahme müssen keine so hohen Anforderungen an die Abmessungen und Eigenschaften der Bohrung 11 (bzw. des Rohres 18) gestellt werden, was die Sicherheit der Ventilfunktion erhöht. Das Vorsehen einer Membran 27 ist natürlich auch bei den oben beschriebenen ersten beiden, sowie auch bei allen weiteren noch zu beschreibenden Beispielen und Ausfuhrungsbeispielen möglich.

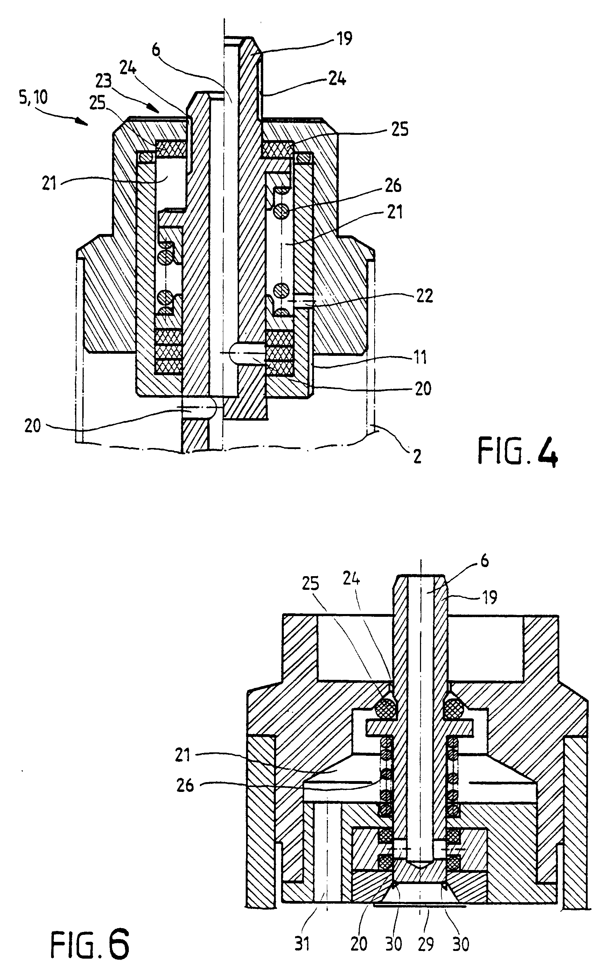

Figur 4 zeigt wiederum ausschnittsweise das Aufsatzteil 10 bzw. den Pumpmechanismus 5 eines weiteren Beispieles einer Sprayeinrichtung. In der linken Hälfte ist der Zustand des Pumpmechanismus 5 und der Ventilanordnung 8 bei Betätigung des Pumpmechanismus dargestellt, während in der rechten Hälfte von Figur 4 der Ruhezustand bei Nicht-Betätigung des Pumpmechanismus gezeigt ist.

Dieses dritte Beispiel unterscheidet sich von der in Figur 3 gezeigten Einrichtung zum einen dadurch, daß für die Dichtung 25 der Lufteintrittsöffnung 24 und für die Abdichtung der Medieneintrittsöffnung 20 in den Medienkanal 6 anstelle von Dichtringen mit im wesentlichen kreisförmigem Querschnitt nun Flachdichtungen verwendet werden. Ein weiterer Unterschied liegt in der Konstruktion der Lufteintrittsöffnung 24. Diese ist nun als Ausnehmung an der Außenwand des Ventilstößels 19 ausgebildet und wird somit zusammen mit dem Herunterdrücken des Ventilstößels 19 bei Betätigung des Pumpmechanismus 5 nach unten geführt. Die Ausnehmung 24 ist in diesem Fall in Längsrichtung des Ventilstößels 19 so groß bemessen, daß sie den Außenraum der Dosier-Sprayflasche 1 mit der Ventilkammer 21 der Ventilanordnung 8 verbindet.

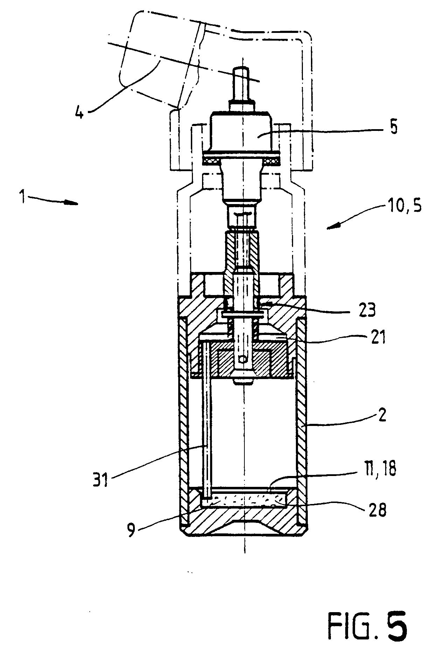

Figur 5 zeigt ein noch weiteres Beispiel einer Dosier-Sprayflasche 1, von der in Figur 6 ausschnittsweise das Aufsatzteil 10 bzw. der Pumpmechanismus 5 im Ruhezustand des Nicht-Betätigens des Pumpmechanismus dargestellt ist.

Dieses vierte Beispiel unterscheidet sich von den oben beschriebenen Einrichtungen dahingehend, daß die Ventilkammer 21 mit einem Rohr 31 verbunden ist, das in eine Kammer 28 am Boden des Aufnahmebehälters 2 mündet. Diese Kammer 28 wiederum ist über eine Bohrung 11, ein Rohr 18 oder eine Membran 27 der oben beschriebenen Art mit dem Innenraum des Aufnahmebehälters 2 verbunden. Hierdurch wird das Einströmen der Luft 9 in Gebrauchsstellung der Sprayflasche 1 (siehe zum Beispiel Figur 1) erleichtert, da die Luft in einen bereits mit Luft 9 gefüllten Bereich des Aufnahmebehälters 2 einströmen kann uind nicht in die Flüssigkeit 3 im Aufnahmebehälter 2 geführt wird.

Eine solche Ausführung einer Dosier-Sprayflasche 1 ist insbesondere auch für die Behandlung von Herzschmerzen durch Nitrolingnat geeignet.

Weiter weist die in Figur 5 und 6 gezeigte Sprayflasche 1 gegenüber den oben beschriebenen Beispielen eine konische Andruckfläche für die Dichtung 25 der Lufteintrittsöffnung 24 auf. Dies ermöglicht eine verbesserte Dichtungsfunktion des Dichtringes 25.

Ferner kann der Aufnahmebehälter 2 vor dem ersten Gebrauch der Dosier-Sprayflasche 1 mit einer flüssigkeitsdichten Membran 29 oder dergleichen versehen sein, so daß der Aufnahmebehälter 2 vor dem ersten Gebrauch absolut dicht ist und die Sprayflüssigkeit 3 sicher im Aufnahmebehälter 2 verbleibt. Diese Membran 29 wird bei Erstgebrauch der Sprayflasche 1 beim Herunterdrücken des Ventilstößels 19 durch eine an diesem angebrachte, geeignete Öffnungsvorrichtung 30 durchstoßen, was den Flüssigkeitseintritt durch die Medieneintrittsöffnung 20 in den Medienkanal 6 ermöglicht. Eine solche Vorrichtung kann selbstverständlich auch bei allen anderen beschriebenen Beispielen und Ausführungsbeispielen vorgesehen sein.

Figur 7 zeigt ein erstes bevorzugtes und in Versuchen erprobtes Ausführungsbeispiel des erfindungsgemäßen Spraymechanismus, wobei die am Boden des Aufnahmebehälters 2 angeordnete Ventilanordnung 8 in der rechten Hälfte von Figur 7A den Zustand bei Betätigung des Pumpmechanismus 5 bzw. während des Sprühvorganges und in der linken Hälfte von Figur 7A bzw. in Figur 7C den Ruhezustand bei Nicht-Betätigung des Pumpmechanismus 5 zeigt. Die Dosier-Sprayeinrichtung 1 befindet sich in Figur 7 in der üblichen Gebrauchsstellung.

Die Dosier-Sprayflasche 1 wird bei Gebrauch zwischen Daumen und Zeigefinger an den beiden Ausbuchtungen 16a und 16b am oberen bzw. unteren Ende der Sprayflasche 1 gehalten. Beim Zusammendrücken der Finger wird die untere Ausbuchtung 16a auf die Betätigungsvorrichtung 7 des Pumpmechanismus 5 gedrückt und ein Herauspumpen der Sprayflüssigkeit 3 aus dem Aufnahmebehälter 2 durch die Medieneintrittsöffnungen 20 befindet sich in Gebrauchsstellung am tiefsten Punkt des Aufnahmebehälters 2, so daß auch die letzten Tropfen der Flüssigkeit 3 angesaugt werden können. Durch die Zerstäuberdüse 4 wird der letzte Rest der Flüssigkeit 3 über die eingebaute Medienleitung 6 abgesaugt. Die Pumpe selbst wird nicht von Flüssigkeit berührt, sondern ist von Luft umgeben.

Die Medienkanäle 32 und 33, welche die Medieneintrittsöffnung 20 mit dem Medienkanal 6 verbinden, sind zum Aufnahmebehälter 2 durch eingesetzte Pfropfen 34 und 35 oder durch entsprechend aufgesetzte Platten 36 im Ultraschweißverfahren verschlossen. Die aufgesetzte Platte 36 zeigt beispielhaft der Ausschnitt von Figur 7B.

Gleichzeitig mit der Betätigung des Pumpmechanismus 5 wird im vorliegenden ersten Ausführungsbeispiel die obere Ausbuchtung 16b, die bei diesem Ausführungsbeispiel von Figur 7 als Scheibe oder Kappe 38 ausgebildet ist, eingedrückt. Hierbei wird das aus Dichtung 55, und Lufteintrittsöffnung 54, bestehende Ventil 53, der am Boden des Aufnahmebehälters 2 angeordneten Ventilanordnung 8 geöffnet und ein Einströmen der Luft von außen durch die Bohrung 11 und den Lufteintrittskanal 37 in den Aufnahmebehälter 2 ermöglicht. Das Einströmen der Luft in der Gebrauchsstellung der Dosier-Sprayflasche 1 erfolgt in den bereits mit Luft 9 gefüllten oben liegenden Bereich des Aufnahmebehälters 2.

Bei der Betätigung auf die obere Ausbuchtung 16b wird die Ventilscheibe 38 gegen den Druck der Feder 39 um einen Hub von etwa 0,5 mm bewegt. Da die Federkraft der Feder 39 geringer ist als die in den Pumpmechanismus 5 eingesetzte Feder, wird bei Druck auf die Ventilscheibe 38 zuerst die Feder 39 zusammengedrückt. Dadurch wird zuerst, d.h. vor Funktion des Pumpmechanimus 5, das Luftventil 53 geöffnet. Deshalb erfolgt der Sprühstoß bei Druck auf den Pumpmechanismus 5 und gleichzeitig strömt eine entsprechende Luftmenge in den Aufnahmebehälter 2 nach. Nach Gebrauch und erfolgtem Sprühstoß schließt sich das Ventil 53 wieder automatisch.

In der Ventilscheibe 38 befindet sich weiter eine Befüllvorrichtung 52 für die Befüllung des Aufnahmebehälters 2 mit Flüssigkeit 3. Die Befüllvorrichtung 52 besteht im wesentlichen aus einer Füllöffnung 40, einem mit einer Feder 42 beaufschlagten Kugelventil 41 und einer Einströmöffnung 43. Bei einem Befüllvorgang auf einem Füllautomaten wird ein Druck auf die Ventilscheibe 38 ausgeübt. Dadurch werden das Kugelventil 41 gegen die Kraft der Feder 42 und gleichzeitig das Luftventil 53 geöffnet, so daß Flüssigkeit 3 durch die Öffnung 43 einströmen und Luft durch den Luftkanal 54, 11, 37, aus dem Aufnahmebehälter 2 ausströmen kann. Nach erfolgter Befüllung schließen sich alle eingerichteten Ventile 53, 41 aufgrund der eingebauten Federn 39, 42 automatisch, wobei die Federn, beispielsweise Druckfedern, einerseits gegen einen beweglichen und andererseits gegen einen ortsfesten Teil anliegen.

In Figur 10 ist nun ein zweites Ausführungsbeispiel einer erfindungsgemäßen Dosier-Sprayflasche 1 in Gebrauchsstellung dargestellt. Dieses Ausführungsbeispiel unterscheidet sich von der oben beschriebenen Sprayeinrichtung 1 von Figur 7 durch verschiedene Merkmale.

Zunächst ist die Lufteintrittsöffnung 44 im Aufsatzteil 10 etwa in einer Linie mit der Zerstäuberdüse 4 angeordnet, wie dies im übrigen auch der Schnittdarstellung von Figur 9 entspricht. Dies ermöglicht gegenüber der Anordnung der Lufteintrittsöffnung 44 in den vorherigen Beispielen und Ausfuhrungsbeispielen eine verbesserte vom Patienten angesogene Luftströmung und damit auch bessere Luftummantelung des Sprühstrahles 46.

Ferner ist gegenüber dem Ausführungsbeispiel von Figur 7 die Anordnung für den Eintritt der Sprayflüssigkeit 3 aus dem Aufnahmebehälter 2 in den Medienkanal 6 des Pumpmechanismus 5 wesentlich vereinfacht. Zu diesem Zweck wird auf der dem Aufnahmebehälter 2 zugewandten Seite des Pumpmechanismus 5 auf diesen eine Umlenkbuchse 47 aufgedrückt und mittels einer umlaufenden Dichtung 48 abgedichtet. Die Umlenkbuchse 47 weist zumindest eine Medieneintrittsöffnung 20 auf, durch die die Sprayflüssigkeit 3 in Gebrauchsstellung der Sprayflasche 1 aus dem Aufnahmenbehälter 2 in den Innenraum der Umlenkbuchse 47 strömen kann. Der Zwischenraum zwischen Umlenkbuchse 47 und Pumpmechanismus 5 bildet Medienkanäle 32 und 33, welche die Medieneintrittsöffnung 20 mit dem Medienkanal 6 verbinden, der durch den Pumpmechanismus 5 und das Aufsatzteil 10 zu der Zerstäuberdüse 4 führt. Durch diese konstruktive Maßnahme können aufwendige Bohrungen an dem den Pumpmechanismus 5 umgebenden Einsatz bzw. Gehäuse entfallen und die Herstellung der Sprayeinrichtung 1 wird vereinfacht. Der Aufnahmebehälter 2 ist in diesem Fall von der bodenseitigen Öffnung her einstückig ausgebildet und kann beispielsweise auf einer einfachen Kunststoffspritzmnaschine hergestellt werden.

Eine weitere Weiterentwicklung dieses zweiten Ausführungsbeispieles liegt bei der im Bodenbereich des Aufnahmebehälters 2 angeordneten Ventilanordnung 8, welche in der linken Hälfte von Figur 10 bei Betätigung des Pumpmechanismus 5 und in der rechten Hälfte von Figur 10 in Ruhestellung dargestellt ist. Als Dichtung 55 des Ventils 53 wird anstelle eines Dichtringes mit im wesentlichen kreisförmigen Querschnitt nun eine Flachdichtung verwendet. Ein weiterer Unterschied findet sich in der Befüllvorrichtung 52 zur Befüllung des Aufnahmebehälters 2 mit Sprayflüssigkeit 3 aus einem Füllautomaten. Die Füllöffnung 40 bleibt vor und während des Befüllvorganges offen. Nach Beendigung des Befüllvorganges fällt eine Kugel 41' auf die Ausbuchtung 16b und rollt über die Schräge der Ventilscheibe 38 über die Füllöffnung 40. Nun wird die Kugel 41' von dem Füllautomat in die Füllöffnung 40 paßdicht eingedrückt, so daß der Aufnahmebehälter 2 flüssigkeitsdicht verschlossen ist.

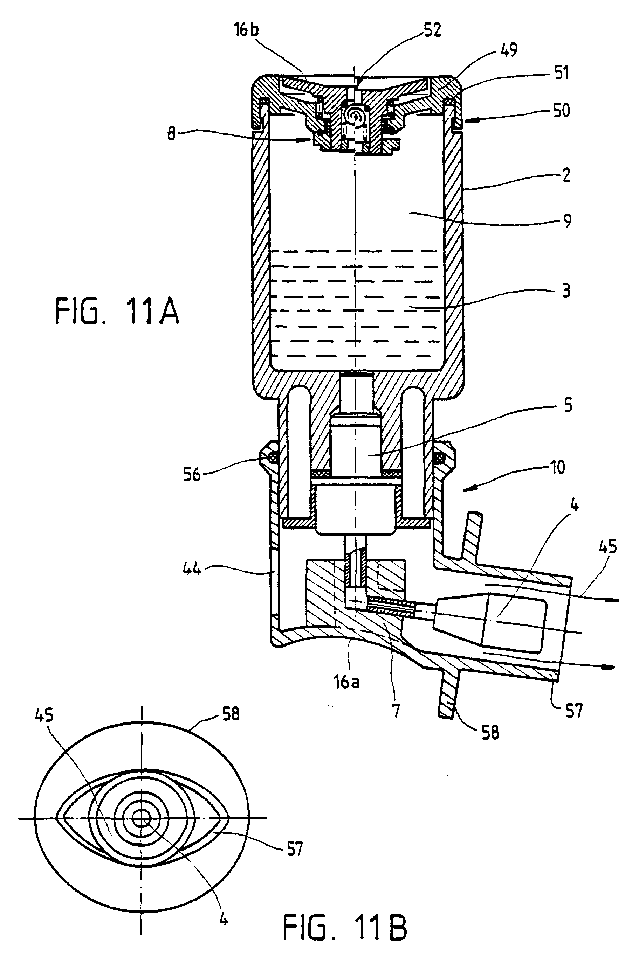

Ein weiteres Ausführungsbeispiel einer Sprayeinrichtung 1 gemäß der vorliegenden Erfindung ist in Figur 11 dargestellt. Die Ventilanordnung 8 und die Befüllvorrichtung 52 sind wie im Ausführungsbeispiel von Figur 7 aufgebaut und am Boden des Aufnahmebehälters 2 angeordnet. Allerdings wird bei der Sprayeinrichtung 1 von Figur 11 der Boden des Aufnahmebehälters 2 durch eine separate Bodenplatte 49 gebildet, die mit dem Aufnahmebehälter 2 in an sich bekannter Weise in Einrastverbindung 50 gebracht und mit Dichtungen 51 abgedichtet werden kann, die zwischen der Bodenplatte 49 und dem Aufnahmebehälter 2 vorgesehen sind.

Ferner schließt in dem in Figur 11A dargestellten Ausführungsbeispiel der Pumpmechanismus 5 mit der Innenwand des Aufnahmebehälters 2, d.h. in Gebrauchsstellung der Sprayflasche 1 mit dem Flüssigkeitsspiegel 3 ab. Außerdem ist das Aufsatzteil 10 mittels einer Dichtung 56, wie beispielsweise eines O-Ringes, dicht auf den Aufnahmebehälter 2 aufgesetzt, so daß vom Patienten ausschließlich Luft durch die Lufteintrittsöffnung 44 im Sprühkopf des Aufsatzteiles 10 angesaugt wird. Ferner weist das Mundstück 57 des Aufsatzteiles 10 einen umlaufenden Mundstückansatz 58 auf, der für den Gebrauch der Sprayeinrichtung 1 bei Kleinkindern etwa die Funktion eines Schnullers übernimmt.

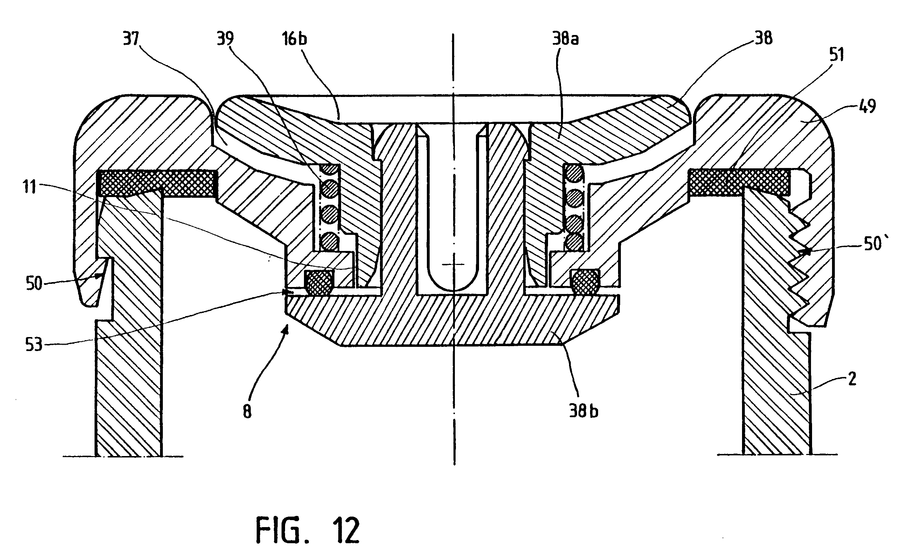

Ein weiteres Ausführungsbeispiel einer erfindungsgemäßen Sprayeinrichtung 1 wird nun anhand von Figur 12 erläutert, wobei in Figur 12 nur der Ausschnitt des Bodenbereiches des Aufnahmebehälters 2 mit zwei Varianten (rechte und linke Hälfte von Figur 12) gezeigt ist.

In diesem vierten Ausführungsbeispiel ist in der Ventilscheibe 38 keine Befüllvorrichtung 52 vorgesehen . Der Aufnahmebehälter 2 wird vielmehr, vorzugsweise von den Pharmafirmen selbst, bei abgenommener Bodenplatte 49 von einem Füllautomaten mit der Sprayflüssigkeit 3 gefüllt. Nach Abschluß des Befüllvorganges wird die Bodenplatte 49 von dem Füllautomaten automatisch mit einer Einrastverbindung 50 (linke Hälfte von Figur 12) auf den Aufnahmebehälter 2 aufgeklipst bzw. aufgeschraubt. Solche Füllautomaten sind bereits auf dem Markt vorhanden. Beim Verschließen des Aufnahmebehälters 2 mit der Bodenplatte 49 wird die Ausbuchtung 16b eingedrückt, so daß Luft durch das Ventil 53, die Bohrung 11 und den Lufteintrittskanal 37 entweichen kann. Die beiden Bauteile 38a und 38b der Ventilscheibe 38 werden vorzugsweise mit Preßsitz zusammengefügt bzw. verklippst.

Anstelle der gezeigten nur einen Luftbohrung 11 können in sämtlichen oben beschriebenen Ausführungsbeispielen natürlich auch mehrere, beispielsweise zwei solcher Bohrungen auf einem Kreisring angeordnet sein, wodurch ein schnelleres Einströmen von Luft in den Aufnahmebehälter 2 erzielt wird.

Ein Vorteil ist es, wenn der Aufnahmebehälter 2 für die Flüssigkeit 3 aus einem durchsichtigen Material gebildet ist. Dadurch kann der Benutzer bzw. der Patient zu jeder Zeit feststellen, ob noch eine Flüssigkeit 3 (lebenswichtiger Wirkstoff) in der Dosier-Sprayeinrichtung 1 zur Verfügung steht. Diese an sich positive Bauweise konnte bei den Dosier-Sprayeinrichtungen nach dem Stand der Technik nicht ausgeführt werden, weil bei aufgebrauchtem Wirkstoff sich in dem Aufnahmebehälter noch FCKW oder andere Treibgase befinden, die dem Patienten eine Füllung mit dem benötigten Wirkstoff vortäuschen. Auch an diesem Merkmal macht sich der wesentliche Vorteil der Dosier-Sprayeinrichtung bemerkbar, bei der für das Austreiben der Flüssigkeit 3 durch den Sprühkopf mit Spraydüse ausschließlich Luft der Umgebung eingesetzt wird.

Statt des gezeigten und beschriebenen Aufnahmebehälters 2 für die Flüssigkeit 3 kann auch eine Patrone aus einem durchsichtigen Werkstoff eingesetzt werden, die mit dem flüssigen Wirkstoff gefüllt ist.

Für den Pumpmechanismus 5 wird eine übliche und auf dem Markt erhältliche Pumpe, beispielsweise die sogenannte Pfeiffer-Pumpe eingesetzt. Der Sprühkopf steht bei dieser Pumpe mit einem Zwischenkolben, einer Manschette und einem Kolben in Verbindfung. Bereits während des ersten Hubes drückt die Manschette Luft in den Pumpenkörper. Bei nach oben gehendem Hub wird im Zylinder ein Vakuum gebildet, welches schließlich die Flüssigkeit 3 aus dem Aufnahmebehälter 2 saugt. Die Dosierpumpe ist mit unterschiedlichen Volumen in den Bereichen von 0,04 bis 1,2 ml erhältlich und im Einsatz.

Die verschiedenen, oben beschriebenen Ausführungsbeispiele lassen sich, wie für den Fachmann leicht zu erkennen, natürlich beliebig kombinieren, um dadurch weitere Ausgestaltungsmöglichkeiten der Dosier-Sprayflasche 1 zu erlangen, die ebenfalls zum Offenbarungsgehalt dieser Anmeldungsunterlagen zählen. Insbesondere können, wie bereits oben angegeben, auch die anhand der Beispiele der Fig. 2-6 erläuterten Merkmale bezüglich der Beschaffenheit der Innenseite der Bohrung/des Rohres und des Vorsehens einer Membran und/oder einer Filtervorrichtung ebenso bei den in den Fig. 7-12 dargestellten Ansführungsbeispielen vorgesehen werden.

## Patentansprüche

1. Spraymechanismus für Sprayflaschen zur dosierten Feinzerstäubung von Flüssigkeiten, mit
einem Aufnahmebehälter (2) zur Aufnahme von Flüssigkeiten (3);
einer Zerstäuberdüse (4) zur Abgabe der Flüssigkeit (3) aus einer Sprayflasche (1);
einem Pumpmechanismus (5) zum Pumpen der Flüssigkeit (3) aus dem Aufnahmebehälter (2) durch einen Medienkanal (6) zu der und durch die Zerstäuberdüse (4) nach außen;
einer Betätigungsvorrichtung (7) zum Betätigen des Pumpmechanismus (5); und
einer Ventilanordnung (8), die eine Luftzufuhr von außen in den Aufnahmebehälter (2) zumindest bei Betätigung des Pumpmechanismus (5) ermöglicht, wobei ein Austritt von Flüssigkeit (3) durch die Ventilanordnung (8) aus dem Aufnahmebehälter (2) bei jedem oder nahezu jedem Außendruck und bei jeder Stellung der Sprayflasche (1) verhindert ist, um einen ständigen Druckausgleich in dem Aufnahmebehälter (2) zu gewährleisten,
**dadurch gekennzeichnet,**
**daß** die Ventilanordnung (8) im Bodenbereich des Aufnahmebehälters (2) vorgesehen ist und eine Öffnung in Form einer Bohrung (11) oder eines Rohres (18) aufweist, die den Innenraum des Aufnahmebehälters (2) mit dem Außenraum der Sprayflasche (1) verbindet und derart bemessen ist, daß ein Austritt der Flüssigkeit (3) durch die Öffnung aus dem Aufnahmebehälter (2) bei jedem oder nahezu jedem Außendruck und bei jeder Stellung der Sprayflasche (1) verhindert ist, wobei die Öffnung (11, 18) mit einer Ventileinrichtung (53) wirkverbunden ist, deren Funktion mit der Betätigung des Pumpmechanismus (5) bzw. mit der der Betätigungsvorrichtung (7) des Pumpmechanismus (5) gekoppelt ist.

2. Spraymechanismus nach Anspruch 1,
**dadurch gekennzeichnet,**
**daß** die Bohrung (11) einen Durchmesser zwischen 0,2 mm und 0,5 mm bei einer Länge von 2 mm bis 10 mm aufweist.

3. Spraymechanismus nach Anspruch 1 oder 2,
**dadurch gekennzeichnet,**
**daß** die Innenseite der Bohrung (11) bzw. des Rohres (18) so beschaffen ist, daß sie von der Flüssigkeit (3) nicht oder nahezu nicht benetzt wird.

4. Spraymechanismus nach einem der Ansprüche 1 bis 3,
**dadurch gekennzeichnet,**
**daß** eine Membran (27) an der Bohrung (11) bzw. dem Rohr (18) vorgesehen ist, die luftdurchlässig ist und den Durchtritt der Flüssigkeit (3) zumindest in Richtung aus dem Aufnahmebehälter (2) heraus sperrt.

5. Spraymechanismus nach einem der vorhergehenden Ansprüche
**dadurch gekennzeichnet,**
**daß** die Bohrung (11) bzw. das Rohr (18) zur Vermeidung von Verschmutzungen oder Verstopfung der Bohrung (11) bzw. des Rohres (18) mit einer Filtervorrichtung versehen ist.

6. Spraymechanismus nach einem der Ansprüche 1 bis 5,
**dadurch gekennzeichnet,**
**daß** die Feder (39) des Ventils (53) weicher ist und bei Betätigung der Dosier-Sprayeinrichtung (1) eher anspricht als die Feder im Pumpmechanismus.

7. Spraymechanismus nach einem der vorhergehenden Ansprüche,
**dadurch gekennzeichnet,**
**daß** im Bodenbereich des Aufnahmebehälters (2) eine Befüllvorrichtung (52) vorgesehen ist.

8. Spraymechanismus nach Anspruch 7,
**dadurch gekennzeichnet,**
**daß** die Befüllvorrichtung (52) eine Füllöffnung (40) und ein Kugelventil (41, 41') zum Verschließen des Aufnahmebehälters (2) aufweist.

9. Spraymechanismus nach Anspruch 8,
**dadurch gekennzeichnet,**
**daß** das Ventil (41) und das Ventil (53) gleichzeitg betätigbar und wirksam sind.

10. Spraymechanismus nach Anspruch 8,
**dadurch gekennzeichnet,**
**daß** der Aufnahmebehälter (2) bei offener Füllöffnung (40) befüllbar und die Füllöffnung (40) nach Abschluß des Befüllvorganges mit dem Kugelventil (41') verschließbar ist.

11. Spraymechanismus nach einem der vorhergehenden Ansprüche,
**dadurch gekennzeichnet,**
**daß** der Aufnahmebehälter (2) eine Bodenplatte (49) aufweist, die mit dem Aufnahmebehälter (2) dicht verbindbar ist.

12. Spraymechanismus nach einem der vorhergehenden Ansprüche,
**dadurch gekennzeichnet,**
**daß** das Aufsatzteil (10) eine Lufteintrittsöffnung (44) aufweist, durch die von dem Patienten bei Gebrauch der Sprayflasche (1) Luft ansaugbar ist.

13. Spraymechanismus nach einem der vorhergehenden Ansprüche,
**dadurch gekennzeichnet,**
**daß** das Mundstück (57) des Aufsatzteiles (10) einen umlaufenden Mundstückansatz (58) mit der Funktion etwa eines Schnullers für Kleinkinder aufweist.

14. Spraymechanismus nach einem der vorhergehenden Ansprüche,
**dadurch gekennzeichnet,**
**daß** der Pumpmechanismus (5) ein mechanischer Pumpmechanismus ist.

15. Spraymechanismus nach einem der vorhergehenden Ansprüche,
**dadurch gekennzeichnet,**
**daß** der Aufnahmebehälter (2) austauschbar mit der Dosier-Sprayflasche (1) verbunden ist.

16. Spraymechanismus nach einem der vorgenannten Ansprüche,
**dadurch gekennzeichnet,**
**daß** der Aufnahmebehälter (2) als Flüssigkeitspatrone ausgebildet ist.

17. Spraymechanismus nach einem der vorgenannten Ansprüche,
**dadurch gekennzeichnet,**
**daß** der Aufnahmebehälter (2) aus einem durchsichtigen Werkstoff gebildet ist.

18. Spraymechanismus nach einem der vorhergehenden Ansprüche,
**dadurch gekennzeichnet,**
**daß** der Spraymechanismus (5) eine Membran (29) oder dergleichen aufweist, die den Aufnahmebehälter (2) flüssigkeitsdicht verschließt und bei dem ersten Gebrauch der Dosier-Sprayflasche (1) durch Betätigen des Pumpmechanismus (5) geöffnet wird, um einen Austritt der Flüssigkeit (3) aus dem Aufnahmebehälter (2) in den Medienkanal (6) zu ermöglichen.

## Claims

1. A spray device suitable for spraying dispensers for allowing metered spraying of liquids, comprising
a storage vessel (2) for storing liquids (3);
a spray nozzle (4) for dispensing said liquid (3) from a spraying dispenser (1);
a pumping mechanism (5) for pumping said liquid (3) from said storage vessel (2) to and through said spray nozzle (4) via a medium passageway (6) to the outside;
an actuating means (7) for actuating said pumping mechanism (5); and
a valve system (8) allowing air to be fed from outside into said storage vessel (2) at least during actuation of said pumping mechanism (5), a delivery of liquid (3) from said storage vessel (2) through said valve system (8) being prevented at every or at least every external pressure and in every position of said spraying dispenser (1) to ensure a steady pressure compensation in said storage vessel (2),
**characterised in that**
said valve system (8) is provided in a bottom area of said storage vessel (2) and comprises an opening in the form of a bore (11) or a tube (18) connecting the inside of said storage vessel (2) to the outside of said spraying dispenser (1) and being dimensioned such that a delivery of liquid (3) from said storage vessel (2) through said opening at every or at least every external pressure and in every position of said spraying dispenser (1) is prevented, wherein said opening (11, 18) is operatingly connected with a valve means (53) the operation of which being coupled to the actuation of said pumping mechanism (5) or said actuating means (7) of said pumping mechanism (5), respectively.

2. The spray device according to claim 1 wherein
said bore (11) has a diameter between 0.2 mm and 0.5 mm and a length of 2 mm to 10 mm.

3. The spray device according to claim 1 or 2 wherein
the inner surface of said bore (11) or tube (18) is constituted such that it will not or almost not be wetted by said liquid (3).

4. The spray device according to anyone of claims 1 to 3 wherein
a membrane (27) is provided at said bore (11) or tube (18) being air permeable and blocking the passage of said liquid (3) at least in the direction out of said storage vessel (2).

5. The spray device according to anyone of preceding claims wherein
said bore (11) or tube (18) for preventing contamination or choking of said bore (11) or tube (18) is provided with a filtering device.

6. The spray device according to anyone of claims 1 to 5 wherein
the spring (39) of said valve means (53) is weaker and upon actuation of said dose spraying dispenser (1) operates sooner than the spring of said pumping mechanism.

7. The spray device according to anyone of preceding claims wherein
a filling means (52) is provided at the bottom area of said storage vessel (2).

8. The spray device according to claim 7 wherein
said filling means (52) comprises a filling opening (40) and a ball valve (41, 41') for sealing said storage vessel (2).

9. The spray device according to claim 8 wherein
said valve (41) and said valve means (53) are simultaneously actuable and operating.

10. The spray device according to claim 8 wherein
said storage vessel (2) can be filled while said filling opening (40) being open and
said filling opening (40) can be closed by said ball valve (41') after completion of the filling operation.

11. The spray device according to anyone of preceding claims wherein
said storage vessel (2) comprises a base plate (49) which may be tightly connected with said storage vessel (2).

12. The spray device according to anyone of preceding claims wherein
the top member (10) comprises an air inlet (44) through which air can be sucked up by a patient during use of said spraying dispenser (1).

13. The spray device according to anyone of preceding claims wherein
a mouth piece (57) of said top member (10) comprises a circumferential mouth piece projection (58) roughly providing the function of a teat for small children.

14. The spray device according to anyone of preceding claims wherein
said pumping mechanism (5) is a mechanical pumping mechanism.

15. The spray device according to anyone of preceding claims wherein
said storage vessel (2) is connected with said dose spraying dispenser (1) in a replaceable manner.

16. The spray device according to anyone of preceding claims wherein
said storage vessel (2) is constructed as a liquid cartridge.

17. The spray device according to anyone of preceding claims wherein
said storage vessel (2) is formed by a transparent material.

18. The spray device according to anyone of preceding claims wherein
said pumping mechanism (5) comprises a membrane (29) or an equivalent element liquid tightly sealing said storage vessel (2) and being opened by actuating said pumping mechanism (5) upon the first use of said dose spraying dispenser to allow the supply of said liquid (3) from said storage vessel (2) into said medium passageway (6).

## Revendications

1. Mécanisme pulvérisateur pour flacons pulvérisateurs pour la pulvérisation fine dosée de liquides avec
un récipient de réception (2) pour recevoir des liquides (3);
une buse de pulvérisation (4) pour délivrer le liquide (3) à partir d'un flacon pulvérisateur (1);
un mécanisme de pompe (5) pour pomper le liquide (3) du récipient de réception (2) par un conduit de substance (6) vers et à travers la buse de pulvérisation (4) vers l'extérieur;
un dispositif d'actionnement (7) pour actionner le mécanisme de pompe (5); et
un dispositif de soupape (8) qui permet une amenée d'air de l'extérieur dans le récipient de réception (2) au moins lors de l'actionnement du mécanisme de pompe (5), une sortie de liquide (3) par le dispositif de soupape (8) hors du récipient de réception (2) étant empêchée pour toute ou presque toute pression extérieure et pour toute position du flacon pulvérisateur (1) pour garantir une compensation de pression permanente dans le récipient de réception (2),
**caractérisé en ce**
**que** le dispositif de soupape (8) est prévu dans la zone du fond du récipient de réception (2) et présente une ouverture en forme de forure (11) ou de tube (18) qui relie l'intérieur du récipient de réception (2) à l'extérieur du flacon pulvérisateur (1) et qui est dimensionnée telle qu'une sortie du liquide (3) par l'ouverture hors du récipient de réception (2) est évitée pour toute ou presque toute pression extérieure et pour toute position du flacon pulvérisateur (1), l'ouverture (11, 18) étant en relation opérationnelle avec un dispositif de soupape (53) dont le fonctionnement est couplé à l'actionnement du mécanisme de pompe (5) ou à celui du dispositif d'actionnement (7) du mécanisme de pompe (5).

2. Mécanisme pulvérisateur selon la revendication 1,
**caractérisé en ce**
**que** la forure (11) présente un diamètre entre 0,2 mm et 0,5 mm pour une longueur de 2 mm à 10 mm.

3. Mécanisme pulvérisateur selon la revendication 1 ou 2,
**caractérisé en ce**
**que** le côté intérieur de la forure (11) ou du tube (18) est constitué de manière telle qu'il n'est pas ou presque pas humidifié par le liquide (3).

4. Mécanisme pulvérisateur selon l'une des revendications 1 à 3,
**caractérisé en ce**
**qu**'une membrane (27), qui est perméable à l'air et qui bloque le passage du liquide (3) tout au moins dans le sens en sortant du récipient de réception (2), est prévue sur la forure (11) ou sur le tube (18).

5. Mécanisme pulvérisateur selon l'une des revendications précédentes,
**caractérisé en ce**
**que** la forure (11) ou le tube (18) est prévu d'un dispositif de filtre pour éviter des impuretés ou pour éviter une obstruction de la forure (11) ou du tube (18).

6. Mécanisme pulvérisateur selon l'une des revendications 1 à 5,
**caractérisé en ce**
**que** le ressort (39) de la soupape (53) est plus souple et répond, lors de l'actionnement du dispositif pulvérisateur doseur (1), plus tôt que le ressort dans le mécanisme de pompe.

7. Mécanisme pulvérisateur selon l'une des revendications précédentes,
**caractérisé en ce**
**qu**'un dispositif de remplissage (52) est prévu dans la zone du fond du récipient de réception (2).

8. Mécanisme pulvérisateur selon la revendication 7,
**caractérisé en ce**
**que** le dispositif de remplissage (52) présente une ouverture de remplissage (40) et une soupape sphérique (41, 41') pour fermer le récipient de réception (2).

9. Mécanisme pulvérisateur selon la revendication 8,
**caractérisé en ce**
**que** la soupape (41) et la soupape (53) peuvent être actionnées simultanément et sont opératives simultanément.

10. Mécanisme pulvérisateur selon la revendication 8,
**caractérisé en ce**
**que** le récipient de réception (2) peut être rempli lorsque l'ouverture de remplissage (40) est ouverte et l'ouverture de remplissage (40) peut être fermée avec la soupape sphérique (41') après achèvement de l'opération de remplissage.

11. Mécanisme pulvérisateur selon l'une des revendications précédentes,
**caractérisé en ce**
**que** le récipient de réception (2) présente une plaque de fond (49) qui peut être reliée de manière étanche au récipient de réception (2).

12. Mécanisme pulvérisateur selon l'une des revendications précédentes,
**caractérisé en ce**
**que** la pièce rapportée (10) présente une ouverture d'entrée de l'air (44) par laquelle l'air peut être aspiré par le patient lors de l'utilisation du flacon pulvérisateur (1).

13. Mécanisme pulvérisateur selon l'une des revendications précédentes,
**caractérisé en ce**
**que** l'embouchure (57) de la pièce rapportée (10) présente un embout d'embouchure périphérique (58) avec la fonction similaire à celle d'une tétine pour enfants en bas âge.

14. Mécanisme pulvérisateur selon l'une des revendications précédentes,
**caractérisé en ce**
**que** le mécanisme de pompe (5) est un mécanisme de pompe mécanique.

15. Mécanisme pulvérisateur selon l'une des revendications précédentes,
**caractérisé en ce**
**que** le récipient de réception (2) est relié en étant échangeable avec le flacon pulvérisateur doseur (1).

16. Mécanisme pulvérisateur selon l'une des revendications précédentes,
**caractérisé en ce**
**que** le récipient de réception (2) est configuré comme une cartouche de liquide.

17. Mécanisme pulvérisateur selon l'une des revendications précédentes,
**caractérisé en ce**
**que** le récipient de réception (2) est formé en une matière première transparente.

18. Mécanisme pulvérisateur selon l'une des revendications précédentes,
**caractérisé en ce**
**que** le mécanisme de pompe (5) présente une membrane (29) ou équivalent qui ferme le récipient de réception (2) de manière étanche au liquide et qui s'ouvre lors de la première utilisation du flacon pulvérisateur doseur (1) en actionnant le mécanisme de pompe (5) pour permettre une sortie de liquide (3) hors du récipient de réception (2) dans le conduit de substance (6).
